(19) Europäisches Patentamt
European Patent Office
Office européen des brevets

(11) **EP 2 737 455 B1**

(12) **EUROPÄISCHE PATENTSCHRIFT**

(45) Veröffentlichungstag und Bekanntmachung des Hinweises auf die Patenterteilung:
**04.11.2015 Patentblatt 2015/45**

(51) Int Cl.:
***G06T 11/00*** *(2006.01)* ***A61B 8/00*** *(2006.01)*

(21) Anmeldenummer: **12775607.0**

(22) Anmeldetag: **27.07.2012**

(86) Internationale Anmeldenummer:
**PCT/DE2012/100228**

(87) Internationale Veröffentlichungsnummer:
**WO 2013/017130 (07.02.2013 Gazette 2013/06)**

(54) **VERFAHREN ZUM AUFFINDEN DER POSITION EINES SCHALLKOPFES**

METHOD FOR DETECTING THE POSITION OF A TRANSDUCER

PROCÉDÉ DE LOCALISATION DE LA POSITION D'UNE SONDE ULTRASONORE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **30.07.2011 DE 102011109037**

(43) Veröffentlichungstag der Anmeldung:
**04.06.2014 Patentblatt 2014/23**

(73) Patentinhaber: **Universität zu Lübeck 23562 Lübeck (DE)**

(72) Erfinder:
• **BRUDER, Ralf**
  **23562 Lübeck (DE)**
• **BRUDER, Gerd**
  **23562 Lübeck (DE)**
• **SCHWEIKARD, Achim**
  **20253 Hamburg (DE)**

(74) Vertreter: **Hansen, Jochen
  Hansen und Heeschen
  Patentanwälte
  Eisenbahnstrasse 5
  21680 Stade (DE)**

(56) Entgegenhaltungen:
**US-A1- 2003 204 139**

• **JENSEN J A ET AL: "Fast simulation of ultrasound images", PROCEEDINGS / 2000 IEEE ULTRASONICS SYMPOSIUM : OCTOBER 22 - 25, 2000, HOTEL CARIBE HILTON, SAN JUAN, PUERTO RICO ; AN INTERNATIONAL SYMPOSIUM, IEEE SERVICE CENTER, PISCATAWAY, NJ, Bd. 2, 22. Oktober 2000 (2000-10-22), Seiten 1721-1724, XP010540945, ISBN: 978-0-7803-6365-6**
• **RAMTIN SHAMS ET AL: "Real-Time Simulation of Medical Ultrasound from CT Images", 6. September 2008 (2008-09-06), MEDICAL IMAGE COMPUTING AND COMPUTER-ASSISTED INTERVENTION Â MICCAI 2008; [LECTURE NOTES IN COMPUTER SCIENCE], SPRINGER BERLIN HEIDELBERG, BERLIN, HEIDELBERG, PAGE(S) 734 - 741, XP019105228, ISBN: 978-3-540-85989-5 das ganze Dokument**
• **AIGER D ET AL: "Real-Time Ultrasound Imaging Simulation", REAL-TIME IMAGING, ACADEMIC PRESS LIMITED, GB, Bd. 4, Nr. 4, 1. August 1998 (1998-08-01), Seiten 263-274, XP004419592, ISSN: 1077-2014, DOI: 10.1006/RTIM.1997.0089**
• **DILLENSEGER J-L ET AL: "Fast simulation of ultrasound images from a CT volume", COMPUTERS IN BIOLOGY AND MEDICINE, NEW YORK, NY, US, Bd. 39, Nr. 2, 1. Februar 2009 (2009-02-01), Seiten 180-186, XP025940785, ISSN: 0010-4825, DOI: 10.1016/J.COMPBIOMED. 2008.12.009 [gefunden am 2009-01-31]**

- **R Bruder, F Ernst, B Stender, L Richter, A Schweikard: "SU-D-220-02: Optimal Transducer Position for 4D Ultrasound Guidance in Cardiac IGRT", Medical Physics, Bd. 38, Nr. 6 31. Juli 2011 (2011-07-31), Seite 3390, XP002688988, DOI: 10.1118/1.3611550 Gefunden im Internet: URL: http://online.medphys.org/resource/1/m phya6/v38/i6/p3390_s1 [gefunden am 2012-12-11]**

**Beschreibung**

Hintergrund der Erfindung:

Strahlenchirurgie ist ein bewährtes Mittel zur Behandlung von Tumorgewebe. Fokussierte, ionisierende Strahlung wird aus verschiedenen Richtungen von außerhalb des menschlichen Körpers auf den Tumor gerichtet. Da die Wirkung im Zielgebiet über eine kumulative Strahlendosis erreicht wird, kann eine Gewichtung mehrerer Strahlen aus verschiedenen Raumwinkeln erfolgen, um umliegendes Gewebe zu schonen und kritische Strukturen besonders zu entlasten. Das CyberKnife (Accuray Inc.) und das Trilogy (Varian Medical Systems) System sind zwei roboterisierte Systeme zur Strahlentherapie.

Moderne Strahlentherapiesysteme besitzen zusätzliche Bildgebungseinrichtungen, um Zielpositionen zu verifizieren und Tumore zu behandeln, die einer respiratorischen Bewegung unterliegen. Auch gibt es Bestrebungen, Zielstrukturen im Bereich des Herzens zu behandeln. Ein Beispiel ist die Behandlung von Vorhofflimmern, bei dem unkoordinierte elektrische Reize die Pumpleistung der Atria stark vermindern und Herzflimmern auslösen. Parallel zu invasiven Katheterablationen wird versucht, durch Strahlung Narbengewebe im Herzen zu erzeugen und auf diese Weise zirkulierende, elektrische Pulse zu unterdrücken.

Die Bewegungsgeschwindigkeit von Zielstrukturen im Bereich des Herzens kann deutlich höher sein als die Geschwindigkeit von Lungentumoren unter Respiration. Da zudem mehrere kritische Strukturen in unmittelbarer Nachbarschaft zum Zielgebiet liegen und ein genaues Patienten-Alignement sichergestellt werden muss, empfiehlt sich während des gesamten Eingriffs eine bildgestützte Überwachung des Zielgebiets und Bewegungskompensation mit hoher Abtastrate.

Ultraschallbildgebung stellt sowohl für die kardiovaskuläre als auch für die herkömmliche Strahlenchirurgie eine schnelle, nicht-ionisierende Alternative zu existierender Röntgenbildgebung dar. Es wurde gezeigt, dass sich die Bewegungsinformation von Zielstrukturen in Ultraschallbildern (etwa durch Pattern Matching) extrahieren lässt. Diese Information kann auf unterschiedliche Weise zur Bewegungskompensation genutzt werden. Die Zielstruktur kann direkt im Ultraschallbild geortet und die Strahlenquelle auf dieses Ziel ausgerichtet, bzw. ihm kontinuierlich nachgeführt werden. Eine Alternative ist die Korrelation zwischen niederfrequent abgetasteter Absolutposition der Zielstruktur (etwa durch Stereo-Röntgenbilder geortete Goldmarker im Zielgebiet) zur schnellen Positionsortung im Ultraschallbild. Auf diese Weise kann eine zeitlich hochaufgelöste Zielposition aus der Ultraschallortung berechnet und zur Nachführung der Strahlenquelle genutzt werden. Die Grundlage für diese Verfahren ist lediglich eine möglichst unverfälschte Ortung der Zielbewegung im Ultraschallbild.

Zur Bewegungserfassung können beliebige, der Untersuchungsregion angepasste Ultraschallsysteme genutzt werden. Zur Visualisierung am Herzen sind z.B. wahlweise transthoraxiale (TTE) oder transoesophageale (TEE) Sonden verfügbar. Die Messdaten (hier durchgängig Ultraschallbilder genannt) können in einer, zwei oder drei Dimensionen erfasst und zur Extraktion von Positionsinformation genutzt werden. Die Sonden können während eines Eingriffs statisch, robotergetragen oder durch Aufkleben auf die Haut auf einer gewählten Schallkopfposition fixiert werden.

Der ausgesandte Ultraschall durchdringt von dieser Position aus das darzustellende Gewebe und ändert dabei - abhängig von den Eigenschaften des durchdrungenen Gewebes - seine Energie und seine Geschwindigkeit. Daraus ergeben sich folgende Probleme:

Eine mögliche Folge dieser Vorgänge ist, dass nicht genug Energie in tieferliegenden Schichten gelangt, um diese abzubilden. Lufteinlagerungen und Knochensubstanz reflektieren oder absorbieren einen Großteil des Schalls und verhindern die Darstellung darunterliegender Gewebsschichten. Besonders im Bereich des Herzens, das von den Lungenflügeln und Rippenbögen verdeckt wird, ist die Suche nach einer geeigneten Schallkopfposition zur Visualisierung einer bestimmten Zielstruktur schwierig. Zudem unterliegen weite Teile des Oberkörpers einer Kombination aus willkürlicher und unwillkürlicher Bewegung (Respiration, Pulsation). Je nach Art und Dauer des zu überwachenden Eingriffs muss eine Visualisierung über den gesamten Behandlungszeitraum gewährleistet sein.

Für eine verlässliche Positionsortung im Ultraschallbild ergeben sich zwei weitere Probleme:

Für eine automatische Ortung und Verfolgung einer Zielstruktur muss diese im Ultraschallbild eine ausreichende Intensität besitzen. Findet in diesem Gebiet nur geringe Reflexion statt oder wird der Ultraschall in einem anderen Winkel als zurück zum Schallkopf reflektiert, kann die Darstellung des Zielgebiets für eine Objektverfolgung ungenügend sein.

Eine von der durchschnittlichen Schallaufzeit im menschlichen Körper abweichende Gewebe-Schallaufzeit hat zur Folge, dass Entfernungen im Ultraschallbild fehlerhaft wiedergegeben werden. Dieser Fehler beträgt bis zu sieben Prozent der Entfernung zwischen Schallkopf und Zielstruktur. Für die Distanz zwischen Schallkopf und Zielstruktur gilt in Abhängigkeit zur Zeit t

$$d_{Gemeesen}(t) = d_{Real}(t) + d_{Messfehler}(t)$$

mit dem von der Entfernung zwischen Schallkopf und Zielstruktur $d_{Real}$ und dem Quotienten aus der im Ultraschallgerät angenommenen Standardschallaufzeit $v_{Standard}$ und der realen Schallaufzeit im Gewebe $v_{Real}$ abhängigen Ortungsfehler

$$d_{Messfehler}(t) = d_{Real}(t) \cdot \frac{v_{Real}(t)}{v_{Standard}}$$

Für den außerhalb des Zielgebiets unbewegten Fall ist $v_{Real}(t)$ = c konstant. Die relative Eigenbewegung der Zielstruktur weist in diesem Fall einen geringen Fehler auf und kann für kleine Eigenbewegungen durch

$$\Delta d_{Gemeesen}(t) = \Delta d_{Real}(t) + \Delta d_{Messfehler}(t)$$
$$\approx \Delta d_{Real}(t)$$

angenähert werden. Zur Nutzung der Absolutposition der Zielstruktur im Ultraschallbild muss der Distanzfehler berechnet werden, was auf verschiedenen Wegen durch Kalibrierung der Größe c, etwa durch positionsbezogene Durchschnittswerte, Simulationsergebnisse oder zusätzliche Ortung wissentlich statischer Strukturen mit bekannter Schallkopfdistanz im Ultraschallbild und den Vergleich von gemessenen und bekannten Entfernungsinformationen, möglich ist.

Unterliegt das Gewebe zwischen Zielstruktur und Schallkopf aber selbst einer Bewegung und kommt es dabei zu einer Änderung der Schallaufzeiten zwischen Zielstruktur und Schallkopf, dann kann die relative Änderung des resultierenden Laufzeitfehlers in Abhängigkeit von der Entfernung $d_{Real}$ in einer ähnlichen Größenordnung wie die Eigenbewegung der Zielstruktur liegen.

$$\Delta d_{Gemeesen}(t) = \Delta d_{Real}(t) + \Delta d_{Messfehler}(t) + d_{Messfehler}(t)$$

Da sich im Bereich des Thorax willkürliche und unwillkürliche Bewegungen überlagern, ist eine Schätzung der auftretenden Fehler extrem schwierig. Sowohl die absolute Positionsinformation der Zielstruktur im Ultraschallbild, als auch die relative Bewegungsinformation sind nicht zur Bewegungskompensation nutzbar.

Stand der Technik:

Bei Hifu (High intensity focused ultrasound) werden in einem aktuellen Forschungsprojekt Gewebeeigenschaften auf Schallgeschwindigkeiten gemappt, um eine saubere Überlagerung möglichst aller eingehenden Ultraschallenergie in einem scharfen Fokuspunkt zu erreichen. Das Ziel ist aber die Zerstörung von Gewebe durch Ultraschall und keine Objektortung.

In der Echokardiographie gibt es Standardpositionen für die Aufnahme von Ultraschallbildern am Herzen (sog. "Schallfenster"), die eine ungehinderte Sicht auf das Herz bei bestimmter Patientenlage und bei angehaltenem Atem ermöglichen. Damit ist zumindest teilweise das Problem der Sichtbarkeit gelöst. In der Strahlentherapie muss ein Patient jedoch zwangsweise auf dem Rücken liegen. Eine Therapiesitzung dauert bis zu 30 Minuten, was ein Luftanhalten erschwert. Die Ziele sind Tumore oder Strukturen am Herzen, die oft auch außerhalb der Standardansichten liegen.

Ferner ist im Stand der Technik die Simulation von virtuellen Ultraschall-Bildern aus Volumendatensätzen beispielsweise aus den Druckschriften Jensen J A et al. "Fast simulation of ultrasound images", Proceedings / 2000 IEEE Ultrasonics Symposium: October 22 - 25, 2000, Hotel Caribe Hilton, San Juan, Puerto Rico; An International Symposium, IEEE Service Center, Piscataway, NJ, Bd. 2, 22. Oktober 2000 (2000-10-22), Seiten 1721-1724, XP010540945, ISBN: 978-0-7803-6365-6, Ramtin Shams et al. "Real-Time Simulation of Medical Ultrasound from CT Images", 6. September 2008 (2008-09-06), Medical Image Computing and Computer-Assisted Intervention a Miccai 2008; (Lecture Notes in Computer Science), Springer Berlin Heidelberg, Berlin, Heidelberg, Page(s) 734-741, XP019105228, ISBN: 978-3-540-85989-5, Aiger D et al. "Real-Time Ultrasound Imaging Simulation", Real-Time Imaging, Academic Press Limited, GB, Bd. 4, Nr. 4, 1. August 1998 (1998-08-01), Seiten 263-274, XP004419592, ISSN: 1077-2014, DOI: 10.2006/RTIM.1997.0089, Dillenseger J-L et al. "Fast simulation of ultrasound images from a CT volume", Computers in Biology and Medicine, New York, NY, US, Bd. 39, Nr. 2, 1. Februar 2009 (2009-02-01), Seiten 180-186, XP025940785, ISSN: 0010-4825, DOI: 10.1016/J.Compbiomed.2008.12.009 und US 2003/204139 A1 (Hashimoto Hiroshi (JP)) bekannt.

Aufgabe der Erfindung:

Der Erfindung liegt damit die Aufgabe zugrunde, ein Verfahren zur ungehinderten Ortung von einer oder mehreren Zielstrukturen im Ultraschallbild zu schaffen. Dabei muss von einer gegebenen Schallkopfposition die Darstellung

- der Zielstruktur(en) in einem definierten Zustand
- der sich (durch Respiration und Pulsation) bewegenden Zielstruktur(en)
- der sich bewegenden Zielstruktur(en) bei gleichzeitiger Bewegung der umliegenden Strukturen bzw. des zwischen Zielstruktur und Schallkopf liegenden Gewebes ermöglicht werden.

Optional soll die von Zielstrukturen gemessene Bewegungsinformation möglichst von Messfehlern aus der zwischen Schallkopf und Zielgebiet stattfindenden Gewebebewegung befreit werden.

Lösung der Aufgabe:

Erfindungsgemäß wird diese Aufgabe durch die Merkmale des Anspruchs 1 gelöst. Die Unteransprüche geben bevorzugte Ausgestaltungen der Erfindung an.

Erfindungsgemäß wird also vorgeschlagen, vor dem Eingriff ein (bevorzugt mehrere) Planungsvolumen (CT oder MRI) des zu schallenden Bereichs zwischen möglichen Positionen des Schallkopfes und der Zielstruktur zu definieren. Sodann werden die akustischen Ultraschallimpedanzen und Ultraschallaufzeiten aus dem bzw. den Planungsbildern klassifiziert. Die optimale Position des Ultraschallkopfes wird anschließend durch Bewertung jeder möglichen Schallkopfposition anhand der ermittelten Größen berechnet und der Ultraschallkopf des überwachenden Ultraschallsystems wird entsprechend positioniert.

Dem im Planungsvolumen dargestellten Gewebe werden seine akustischen Eigenschaften (Schallgeschwindigkeit, akustische Impedanz) zugeordnet. Die Zuordnung kann z.B. anhand der räumlichen Lage (Klassifikation segmentierter Bereiche) oder durch Nutzung einer geeigneten Transferfunktion zwischen Intensitätswerten im Planungsvolumen und akustischen Eigenschaften erfolgen.

Aufgrund dieser Eigenschaften wird von jeder möglichen Schallkopfposition aus die Sicht auf die Zielstruktur(en) simuliert. Dies kann auf unterschiedliche Weise erfolgen: Komplexe Ultraschallsimulatoren (siehe US 7,835,892, US 7,731,499 und US 7,699,778) erstellen ein virtuelles Ultraschallbild aus dem Planungsvolumen. Als Sichtbarkeitskriterium der Zielstruktur können in dem generierten Ultraschallbild u. A. die Helligkeit (Reflektion) oder die Entropie im Zielgebiet ausgewertet werden. Alternativ können die Gewebeabsorption für den in direkter Sichtverbindung zwischen Schallkopf und Zielstruktur laufenden Schall und die Reflektion des Schalls im Zielgebiet zur Approximation der Sichtbarkeit der Zielstruktur genutzt werden. Neben der Sichtbarkeit der Zielstruktur wird die Schallaufzeit zwischen Zielstruktur(en) und Schallkopf anhand der Daten im Planungsvolumen simuliert.

Ist der zu erwartende Aufenthaltsbereich einer Zielstruktur in einem Planungsvolumen gegeben, dann werden Sicht und Schallaufzeit für je eine Schallkopfposition zu jeder möglichen Zielposition im Aufenthaltsbereich simuliert. Sind mehrere Volumendatensätze für verschiedene Bewegungszustände von Zielstruktur und umliegendem Gewebe vorhanden, dann wird die Berechnung parallel auf allen Planungsvolumen durchgeführt.

Zur Minimierung des Messfehlers durch Gewebebewegung werden die simulierten Schallaufzeiten abhängig von den verfügbaren Planungsdatensätzen und der Messaufgabe analysiert. Kriterien sind

- die zu erwartende Abweichung der Schallaufzeit von der im Ultraschallgerät angenommenen Standardlaufzeit,
- die Änderung der Schallaufzeit zu einer Zielstruktur für die zu erwartende Zielbewegung im Planungsvolumen,
- die Änderung der Schallaufzeit zu einer Zielstruktur für mehrere Planungsvolumen,
- die Differenz der Schallaufzeiten zu mehreren Zielstrukturen im Ultraschallbild.

Ein Algorithmus wählt anhand der gegebenen Sichtbarkeiten und Schallaufkriterien eine oder mehrere Schallkopfpositionen aus. (Der Schallkopf wird auf diese Position gesetzt).

Ein Beispiel ist der Einsatz des Verfahrens zur Positionierung eines zur Bewegungskompensation in der roboterisierten, bildgestützten Strahlentherapie (IGRT) genutzten Ultraschallkopfes. Die Aufgabe ist die lückenlose Verfolgung einer Struktur (Tumor, Behandlungsgebiet) im Bereich des menschlichen Thorax, die respiratorischer und/oder pulsatorischer Bewegung unterliegen kann.

Als Vorbereitung auf den Behandlungsschritt werden üblicherweise ein oder mehrere CT-Planungsvolumen erstellt, die den Thorax in verschiedenen Atmungszuständen oder Herzphasen abbilden. Auf ihnen erfolgt die Planung des strahlenchirurgischen Eingriffs durch Segmentierung von Ziel- und Risikostrukturen und die Optimierung der Gewichtung einer Menge möglicher Strahlensätze aus verschiedenen Richtungen auf das Zielgebiet.

In diesen Vorverarbeitungsschritt gliedert sich das hier beschriebene Verfahren ein. Auf Basis des/der CT Volumendaten werden mögliche Kontaktflächen zur Auflage des Schallkopfes, etwa durch Extraktion der Hautoberfläche bestimmt. Die verschiedenen Positionen werden sodann einer Bewertung unterzogen, inwieweit sie sich zur Beobachtung einer Zielstruktur im Thorax-Inneren mittels Ultraschall eignen.

Dazu wird zunächst die Sichtbarkeit einer zur Bewegungserfassung genutzten Zielstruktur überprüft.

Tabelle 1: Zuordnung von Hounsfield-Werten zu Schalleigenschaften

| Gewebe | Hounsfield-Einheiten (min/max) | Schalllmpedanz (kg(m$^2$/s) | Schallgeschwindigkeit (m/s) |
|---|---|---|---|
| Reine Luft | -1000 | 0,0004 | 331 |
| Lunge | -800/-500 | 0,003 | 331 |
| Fettgewebe | -100/10 | 0,138 | 1468 |
| Wasser | -10/10 | 1,53 | 1526 |
| Leber | 40/60 | 1,65 | 1559 |
| Knochen | 250/1000 | 6,66 | 3600 |
| Blut | 30/70 | 1,60 | 1562 |
| Herzgewebe | 20/50 | 1,67 | 1590 |

Tabelle 1 gibt einen Überblick über die Gewebe im Bereich des Herzens mit den dazu gehörenden typischen Intervallen der in der CT messbaren Hounsfield Einheiten wieder. Den verschiedenen Materialien werden ihre durchschnittlichen, akustischen Eigenschaften (akustische Impedanz, Schallgeschwindigkeit, etc.) gegenübergestellt. Mit Hilfe dieser Daten werden den Voxeln des Planungsvolumens die akustischen Eigenschaften der dargestellten Anatomie zugeordnet.

Die Bewertung der Zielsichtbarkeit erfolgt im Rahmen eines vereinfachten Modells für die Schallabsorption im Gewebe, bei dem das Planungsvolumen vom Ultraschallkopf zur Zielstruktur auf einer direkten Verbindungslinie durchlaufen und dabei die Absorption des ausgesandten Schalls berechnet wird. Vereinfacht können Reflexion und Streuung als Hauptanteile der Absorption aus den Hounsfield-Einheiten der auf diesem Weg liegenden Volumenvoxel berechnet und aufintegriert werden. Weitere - die Absorption des Strahls in der Regel beeinflussende - Faktoren wie z.B. Interferenz und Brechung werden in diesem Modell ignoriert.

Auf diese Weise wird zu jeder möglichen Schallkopfposition die Zielsichtbarkeit für alle Positionen der Zielstruktur über alle Planungsvolumen als der durch Absorption verminderte Anteil des die Zielstruktur erreichenden Ultraschalls definiert.

Sind zu der jeweiligen Schallkopfposition mehrere Zielpositionen oder Planungsvolumen vorhanden, wird die Zielsichtbarkeit für die Schallkopfposition als das Minimum der einzelnen Zielsichtbarkeiten berechnet.

Eine optimale Schallkopfposition kann durch Optimierung der Zielsichtbarkeit über alle Schallkopfpositionen gefunden werden. Weiterhin wird ein Schwellwert für eine akzeptable Sichtbarkeit genutzt und alle Schallkopfpositionen mit Sichtbarkeiten über diesem Schwellwert werden zur weiteren Verarbeitung ausgegeben.

Einer dieser Verarbeitungsschritte ist die Minimierung der durch Gewebebewegung zwischen Schallkopf und Zielstruktur induzierten, zeit- und positionsabhängigen Entfernungsfehler in der Positionsmessung der Zielstruktur. Hierzu wird in einem zweiten Optimierungsschritt unter allen Schallkopfpositionen mit ausreichender Zielsichtbarkeit diejenige Position mit dem geringsten, zu erwartenden Entfernungsfehler ausgewählt. Parallel zur Bestimmung der Absorption wird die Schallaufzeit auf der direkten Verbindungslinie zwischen Schallkopf und Zielstruktur bestimmt. Abhängig von der Messaufgabe ergeben sich folgende Optimierungsaufgaben:

- Zur absoluten Positionsmessung von Zielstrukturen muss die Differenz zwischen Standard-Schallgeschwindigkeit und der aus den Gewebeeigenschaften berechneten, realen Schallaufzeit minimiert werden. Als Fehlerfunktion kann hier der RMS-Fehler der Geschwindigkeitsunterschiede auf der direkten Verbindungslinie zwischen Schallkopf und Zielstruktur genutzt werden. Eine Minimierung dieser Funktion liefert die optimale Schallkopfposition.

- Soll eine relative Bewegungsinformation der Zielstruktur, etwa zur Korrelation, gewonnen werden, muss die Änderung der Schallaufzeit zwischen Schallkopf und Zielstruktur minimiert werden. Über alle Zielpositionen und Planungsvolumen wird der RMS-Fehler zwischen errechneter Schallaufzeit und mittlerer, errechneter Schallaufzeit als Fehlerfunktion definiert und minimiert.

Anschließend wird der Ultraschallkopf auf der errechneten Position platziert.

Die im Stand der Technik bekannten Verfahren unterscheiden sich im Wesentlichen von der diesseitigen Erfindung durch:

- die Verwendung eines fokussierten US als Therapiewerkzeug und
- die fortlaufende Beobachtung während des Eingriffs mit MR sowie
- die Verwendung eines US-Array.

Die diesseitige Erfindung dient dagegen der Bildgebung, wohingegen MRT oder CT der Planung vor dem Eingriff dienen. Und insbesondere ist die Verwendung nur eines Ultraschallkopfes als Besonderheit herauszustellen.

**Patentansprüche**

1. Verfahren zum Auffinden der Position eines Schallkopfes zur Positions- und Bewegungsüberwachung einer oder mehrerer Zielstrukturen zur Vorbereitung oder während eines Eingriffs, mit den Schritten:

   - paralleles Simulieren virtueller Ultraschall-Bilder aus einer Mehrzahl von Volumendatensätzen (CT/MRT) für verschiedene Bewegungszustände der Zielstruktur oder der Zielstrukturen und umliegendem Gewebe für jede mögliche vorgewählte Schallkopfposition auf einer möglichen Kontaktfläche,
   - Ermitteln der Zielsichtbarkeit als den durch Absorption verminderten Anteil des die Zielstruktur erreichenden Ultraschalls oder als das Minimum des durch Absorption verminderten Anteils des die Zielstrukturen erreichenden Ultraschalls für alle simulierten Ultraschall-Bilder,
   - Positionieren des Ultraschallkopfes auf der Kontaktfläche in einer Schallkopfposition, für die die Zielsichtbarkeit über einem Schwellwert für eine akzeptable Sichtbarkeit liegt, und die durch Minimierung der durch Gewebebewegung zwischen Schallkopf und Zielstruktur oder Zielstrukturen induzierten, zeit- und positionsabhängigen Entfernungsfehler in der Positionsmessung der Zielstruktur oder Zielstrukturen bestimmt wird.

2. Verfahren nach Anspruch 1, mit den Schritten:

   - Zuordnen von Ultraschalleigenschaften wie Schallgeschwindigkeit und Schallimpedanz zu Strukturen aus dem Volumendatensatz durch eine lokale Funktion der Intensitätswerte in dem Volumendatensatz oder die Segmentierung unterschiedlicher Schalleigenschaften im Volumendatensatz und Zuordnung der Schalleigenschaften zu den segmentierten Bereichen,
   - Ermitteln einer oder mehrerer Schallkopfpositionen unter allen möglichen Schallkopfpositionen an der oder denen Reflexionen und Absorptionen des zwischen Schallkopf und Zielstruktur oder Zielstrukturen liegenden Gewebes die Einbringung der höchsten Schallintensität bzw. einer Mindestschallintensität in die Zielstruktur oder Zielstrukturen, und damit eine Mindestdarstellungsqualität im Ultraschall-Bild, erlauben.

3. Verfahren nach Anspruch 2, mit dem Schritt:

   Berechnen der optimalen Schallkopfposition bei einer Bewegung der Zielstruktur oder Zielstrukturen oder der der Zielstruktur oder Zielstrukturen vorgelagerter Strukturen aus dem Minimum der einzelnen, in mehreren Volumendatensätzen und/oder zu mehreren Positionen der Zielstruktur berechneten Schallintensitäten.

4. Verfahren nach Anspruch 2 oder 3, mit dem Schritt:

   Wählen der optimalen Schallkopfposition aus den berechneten möglichen Schallkopfpositionen mit Mindestschallintensität, bei der sich die Schallaufzeiten der vorgelagerten Strukturen zwischen Schallkopfposition und Zielstruktur oder Zielstrukturen über die Zeit möglichst wenig ändert.

5. Verfahren nach einem der Ansprüche 2 bis 4, mit dem Schritt:

   Wählen einer optimalen Schallkopfposition aus den berechneten möglichen Schallkopfpositionen mit Mindestschallintensität, bei der sich die Schallaufzeiten zwischen der Schallkopfposition und den einzelnen Zielstrukturen untereinander möglichst wenig unterscheiden.

## Claims

1. A method for finding the position of a transducer for monitoring the position and motion of one or more target structures for preparation prior to or during an operation, with the steps

   - parallel simulation of virtual ultrasound images from a plurality of volume data sets (CT/MRT) for different states of motion of the target structure or the target structures and surrounding tissue for any possible preselected transducer position on a possible contact area,
   - determining the target visibility as the minimum of the absorption-attenuated proportion of the ultrasound reaching the target structure or as the minimum of the absorption-attenuated proportion of the ultrasound reaching the target structure for all simulated ultrasound images,
   - positioning the ultrasonic transducer head on the contact surface in a transducer position, for which the target visibility is above a level for an acceptable visibility and which is determined by minimization of the tissue movement between transducer und target structure or target structures induced time- and positiondepending distance error in the measurement of the position of the target structure or the target structures.

2. The method according to claim 1, with the steps

   - correlating ultrasonic-properties such as speed of sound and acoustic impedance to the structures of the volume-data-set with a local function of the intensity-values in the volume-data-set or the segmentation of different acoustic properties in the volume-data-set and correlating the sound characteristics to the segmented regions,
   - determining one or more transducer positions from among all possible transducer positions where a or the reflections and absorptions between transducer and the target structure- or target structures-beneath tissue allow the insertion of the highest sound intensity, or the minimum sound intensity in the target structure or the target structures, so that a minimum of image quality in the ultrasonic imaging could be achieved.

3. The method according to claim 2, with the step:

   calculating the optimal transducer position considering movement of the target structure or target structures or the target structure or target structures, or the upstream to a or the target structures located structures, with the minimum of the individual, in a plurality of volume-data-sets and/or for several positions of the target structure calculated sound intensities.

4. The method according to claim 2 or 3, with the step:

   selecting the ideal transducer position from the calculated potential transducer positions with minimum sound intensity, where the sound propagation times of the upstream located structures between transducer positions and target structure or target structures changes preferably only a little over time.

5. The method according to any one of claims 2 to 4, with the step:

   selecting the ideal transducer position from the calculated potential transducer positions with minimum sound intensity, where the sound propagation times between transducer position and the target structures differ as little as possible from each other.

## Revendications

1. Procédé de localisation de la position d'une sonde à ultrasons pour la surveillance de la position et des mouvements d'une ou de plusieurs structures cibles afin de préparer une intervention, ou pendant celle-ci, comprenant les étapes :

   - de simulation parallèle d'images ultrasonores virtuelles à partir d'une pluralité d'ensembles de données volumiques (CT/MRT) pour différents états de mouvement de la structure cible ou des structures cibles et du tissu environnant pour chaque position possible présélectionnée de la sonde à ultrasons sur une surface de contact possible,

- de détermination de la visibilité de la cible comme part réduite par absorption de l'onde ultrasonore atteignant la structure cible ou comme le minimum de la part réduite par absorption de l'onde ultrasonore atteignant les structures cibles pour toutes les images ultrasonores simulées,

- de positionnement de la sonde à ultrasons sur la surface de contact dans une position de sonde à ultrasons pour laquelle la visibilité de la cible est supérieure à une valeur seuil pour une visibilité acceptable, et qui est définie en réduisant au minimum des erreurs de distance qui dépendent du temps et de la position et qui sont induites par le mouvement du tissu entre la sonde à ultrasons et la structure cible ou les structures cibles, lors de la mesure de la position de la structure cible ou des structures cibles.

2. Procédé selon la revendication 1,
   comprenant les étapes :

   - d'affectation de propriétés ultrasonores telles que la vitesse du son et l'impédance acoustique aux structures de l'ensemble de données volumiques au moyen d'une fonction locale des valeurs d'intensité dans l'ensemble de données volumiques ou la segmentation de différentes propriétés acoustiques dans l'ensemble de données volumiques et affectation des propriétés acoustiques aux zones segmentées,
   - de détermination d'une ou de plusieurs positions de la sonde à ultrasons parmi toutes les positions possibles de la sonde à ultrasons dans laquelle ou dans lesquelles des réflexions et absorptions du tissu situé entre la sonde à ultrasons et la structure cible ou les structures cibles permettent l'introduction de l'intensité acoustique maximale ou d'une intensité acoustique minimale dans la structure cible ou les structures cibles, et donc une qualité de représentation minimale dans l'image ultrasonore.

3. Procédé selon la revendication 2,
   comprenant l'étape :

   de calcul de la position optimale de la sonde à ultrasons lors d'un mouvement de la structure cible ou des structures cibles ou des structures situées en amont de la structure cible ou des structures cibles à partir du minimum des différentes intensités acoustiques calculées dans plusieurs ensembles de données volumiques et/ou pour plusieurs positions de la structure cible.

4. Procédé selon la revendication 2 ou 3,
   comprenant l'étape :

   de sélection de la position optimale de la sonde à ultrasons à partir des positions possibles calculées de la sonde à ultrasons présentant l'intensité acoustique minimale, dans laquelle les temps de parcours du son des structures situées en amont entre la position de la sonde à ultrasons et la structure cible ou les structures cibles varient le moins possible au fil du temps.

5. Procédé selon l'une des revendications 2 à 4,
   comprenant l'étape :

   de sélection d'une position optimale de la sonde à ultrasons à partir des positions calculées possibles de la sonde à ultrasons présentant une intensité acoustique minimale, dans laquelle les temps de parcours du son entre la position de la sonde à ultrasons et les différentes structures cibles se distinguent le moins possible les uns des autres.

**IN DER BESCHREIBUNG AUFGEFÜHRTE DOKUMENTE**

**In der Beschreibung aufgeführte Patentdokumente**

- US 2003204139 A1
- US 7835892 B
- US 7731499 B
- US 7699778 B

**In der Beschreibung aufgeführte Nicht-Patentliteratur**

- **JENSEN J A et al.** Fast simulation of ultrasound images. *Proceedings / 2000 IEEE Ultrasonics Symposium,* 22. Oktober 2000
- **HOTEL CARIBE HILTON ; SAN JUAN ; PUERTO RICO.** An International Symposium. IEEE Service Center, 22. Oktober 2000, vol. 2, 1721-1724
- Real-Time Simulation of Medical Ultrasound from CT Images. **RAMTIN SHAMS et al.** Medical Image Computing and Computer-Assisted Intervention a Miccai 2008. Springer, 06. September 2008, 734-741
- Real-Time Ultrasound Imaging Simulation. **AIGER D et al.** Real-Time Imaging. Academic Press Limited, 01. August 1998, vol. 4, 263-274
- **DILLENSEGER J-L et al.** Fast simulation of ultrasound images from a CT volume. *Computers in Biology and Medicine,* 01. Februar 2009, vol. 39, ISSN 0010-4825, 180-186